# EUROPEAN PATENT APPLICATION

(11) **EP 4 216 225 A1**
(43) Date of publication of application: **26.07.2023**
(21) Application number: 22152443.2
(22) Date of filing: 20.01.2022
(51) Int. Cl.: G16H 10/20, G16H 30/40, G16H 50/30

(54) **COGNITIVE IMPAIRMENT DETERMINATION APPARATUS**

(71) Applicant: Koninklijke Philips N.V., 5656 AG Eindhoven (NL)
(72) Inventor: HART DE RUIJTER-BEKKER, Evelijne Machteld, Eindhoven (NL); VAN EE, Raymond, Eindhoven (NL); KLAMING, Laura, Eindhoven (NL); LAMERICHS, Rudolf Mathias Johannes Nicolaas, Eindhoven (NL); DENISSEN, Adrianus Johannes Maria, Eindhoven (NL)
(74) Representative: Philips Intellectual Property & Standards

(57) **Abstract**

The present invention relates to a cognitive impairment determination apparatus (10), comprising: an input unit (20); a processing unit (30, 120); and an output unit (40). The input unit is configured to provide the processing unit with a plurality of first task trial results, wherein the plurality of first task trial results is generated by a patient having conducted a plurality of trials of a first task. The processing unit is configured to determine an index for the patient, wherein the determination of the index comprises a calculation of a representation of fluctuations in the plurality of first task trial results. The processing unit is configured to determine that the patient should undergo a scan of the brain, wherein the determination comprises utilization of the index for the patient. The output unit is configured to output information that the patient should undergo the scan of the brain if the processing unit determines that the patient should undergo the scan of the brain.

## Description

### FIELD OF THE INVENTION

The present invention relates to a cognitive impairment determination apparatus, a cognitive impairment determination system, and cognitive impairment determination methods, as well as to a computer program element and a computer readable medium.

### BACKGROUND OF THE INVENTION

Traumatic brain injury (TBI) constitutes a large, and rapidly growing public health concern. Each year, around 69 million people worldwide sustain a TBI. After arrival at the hospital, a CT scan is made if the neurologist suspects the presence of a life-threatening condition, in particular haemorrhagic bleedings. If no gross abnormalities are found, patients are sent home with the diagnosis mild TBI (mTBI). There is no further monitoring or treatment. Most traumatic brain injuries (TBIs) are classified as mild. (>85%) Symptoms are assumed to be *transient* and *non-neurological.* Both assumptions are incorrect.

As for transience, around 50% of mTBI patients develop chronic problems., They may exhibit persistent behavioral, emotional, and especially cognitive symptoms. Patients may be referred to specialized rehabilitation clinics where diagnostic cognitive tests are administered and rehabilitation therapy is provided, but no further brain scans are made. Even in specialized rehabilitation clinics, cognitive problems may easily be overlookedThe gold standard for assessing the degree of cognitive impairment in clinical practice involves paper-and-pencil neuropsychological tests. Most patients with subtle cognitive deficits, such as mTBI patients, do not show profound abnormalities on these standardized tests despite significant subjective complaints of cognitive dysfunction in daily life. If performance meets norms, patients are considered unimpaired. However, standardized tests are inadequate for diagnosis of subtle cognitive deficits because dependent measures (such as reaction times and error rates) merely reflect the end-result of a diversity of underlying cognitive processes. In other words, test outcomes inform us on whether patients were able to complete a task, not on how they have completed a task. Importantly, for neuropsychological tests that consist of several trials (e.g. reaction time tasks or cancellations tests), test scores are typically derived by averaging measures collected over a large set of trials. This means that crucial information on fluctuations in performance over time, which is indicative of subtle cognitive impairment and has been found to correlate with complaints of daily life dysfunction (Spreij et al), gets lost.

As for the non-neurological nature of symptoms, when patients do not recover (fully), a routine MRI is sometimes made. It demonstrates brain damage in ∼ 30% of patients with clean CTs. The use of more advanced MRI techniques (fMRI, DTI, MWI) - that are better suited to detect the subtle brain abnormalities characteristic of mTBI (primarily diffuse axonal injury) -increases the percentage of patients with identifiable brain damage (see: Yin B, Li DD, Huang H, Gu CH, Bai GH, Hu LX, Zhuang JF, Zhang M.Yin B, et al. Longitudinal Changes in Diffusion Tensor Imaging Following Mild Traumatic Brain Injury and Correlation With Outcome. Front Neural Circuits. 2019 May 7;13:28, and Bai L, Bai G, Wang S, et al. Strategic white matter injury associated with long-term information processing speed deficits in mild traumatic brain injury. Hum Brain Mapp. 2020). Although the added value of advanced imaging for mTBI diagnosis has clearly been demonstrated in the lab, the use of such techniques has not yet found its way to the clinic. This is because introducing (advanced) MRI scanning for mTBI diagnosis is time-consuming, involves extra costs (as MRI scans are not routinely made today), requires trained staff, and may be redundant in some cases (for those patients that will recover spontaneously). It is to be noted that other advanced imaging techniques such as the utilization of ASL, or PET can also or alternatively to MRI be used to detect the subtle brain abnormalities characteristic of mTBI.

There is a need to resolve these issues.

### SUMMARY OF THE INVENTION

It would be advantageous to provide a technique to assess when an advanced image technique (such as a structural MRI scan, a functional fMRI scan, an ASL scan, or a PET scan) of a patient's brain should be undertaken. The object of the present invention is solved with the subject matter of the independent claims, wherein further embodiments are incorporated in the dependent claims. It should be noted that the following described aspects and examples of the invention apply to the cognitive impairment determination apparatus, the cognitive impairment determination system, the cognitive impairment determination methods, as well as to the computer program element and the computer readable medium.

In a first aspect, there is provided a cognitive impairment determination apparatus. The apparatus comprises:
- an input unit;
- a processing unit; and
- an output unit.

The input unit is configured to provide the processing unit with a plurality of first task trial results, wherein the plurality of first task trial results is generated by a patient having conducted a plurality of trials of a first task. The processing unit is configured to determine an index for the patient, wherein the determination of the index comprises a calculation of a representation of fluctuations in the plurality of first task trial results. The processing unit is configured to determine that the patient should undergo a scan of the brain, wherein the determination comprises utilization of the index for the patient. The output unit is configured to output information that the patient should undergo the scan of the brain if the processing unit determines that the patient should undergo the scan of the brain.

In this manner it can be determined if a patient should undergo a costly scan such as a MRI or PET scan on the basis of fluctuations in performance how they carry out a task. Thus, if a patient is suspected of having a traumatic brain injury (TBI) or a mild traumatic brain injury (mTBI) they can as soon as possible be provided with a task to do a number of times (here termed trials of the task), and the variability in the results can be used as an indicator that the patient could indeed have a brain injury and should have an MRI scan to determine if there is an injury and the extent of that injury. Alternatively, the (trials of the) task can be repeated over an extended period, for example daily or weekly or monthly and again the variability in the results can be used as an indicator that there is some neurological impairment, for example caused by MS or dementia and the patient could then have an MRI scan to further quantify this.

In an example, each first task trial result of the plurality of first task trial results comprises a time for the patient to conduct the associated trial of the first task.

Thus, a variability in time a patient takes to complete each trial of multiple trials of a task can be used to determine if the patient should have an MRI scan. In other words, rather than an average time associated with trials of a task, the variability or fluctuations in the time taken to carry out each trial of the trials of the task can be used as an indicated that the patient could have a brain injury and that the patient should have an MRI scan to further quantify that.

Thus, regarding the example task of pressing a button when a red dot is presented on a screen (the task) the instructions associated with the task is "press the button as quickly as you can only when you see a red dot" and the trial results in this case comprise the times between the red dot being presented and the time when the button was pressed for the multiple times the red dot was presented on the screen. The fluctuation in the plurality of trials results then comprises the fluctuations in the times between the red dot being shown and the patient pressing the button, and the index for the patient can then comprise this fluctuation information.

In an example, the calculation of the representation of fluctuations in the plurality of first task trial results comprises calculation of a standard deviation associated with the plurality of first task trial results.

Thus, for example regarding the example of the patient being asked to press a button when a dot is shown on a screen and a reaction time between the dot being shown and the button is pressed are trials of a task, there could be for example 400 trials. This the enables variations/standard deviation of the patient's response time to be determined.

In an example, each trial of the plurality of trials of the first task was conducted on the same day.

In an example, at least one trial of the plurality of trials of the first task was conducted on a different day to another trial of the plurality of trials of the first task.

In an example, the input unit is configured to provide the processing unit with a plurality of second task trial results, wherein the plurality of second task trial results is generated by the patient having conducted a plurality of trials of a second task, different to the first task, and wherein the determination of the index comprises a calculation of a representation of fluctuations in the plurality of second task trial results.

In this manner, not only can the variability or fluctuations in the performance of one task be used to determine if the patient is potentially cognitively impaired, but variability across more than one task can help to better make that determination.

In an example, the determination of the index comprises utilization of a first weighting factor applied to the representation of fluctuations in the plurality of first task trial results and a second weighting factor applied to the representation of fluctuations in the plurality of second task trial results.

Thus for example, a standard deviation associated with times taken to conduct individual trials of a first task can be multiplied by a first weighting factor and a standard deviation associated with times taken to conduct individual trials of a second task can be multiplied by a second weighting factor. By weighting these separate indexes a (sub)index can be calculated that can better indicate if the patient should have a scan or not. The weighting factors can for example be predetermined.

In an example, the determination that the patient should undergo the scan of the brain comprises a comparison of the index for the patient with a threshold index value.

In an example, the threshold is determined from normalization tables and/or expert input.

In a second aspect, there is provided a cognitive impairment determination system. The system comprises:
- a task interaction unit;
- a processing unit; and
- an image acquisition unit (130).

The task interaction unit is configured to present a plurality of trials of a first task to a patient and acquire a plurality of first task trial results generated by the patient having conducted the plurality of trials of the first task. The task interaction unit is configured to provide the plurality of first task trial results to the processing unit. The processing unit is configured to determine an index for the patient, wherein the determination of the index comprises a calculation of a representation of fluctuations in the plurality of first task trial results. The processing unit is configured to determine that the patient should undergo a scan of the brain, wherein the determination comprises utilization of the index for the patient. The image acquisition unit is configured to carry out a scan of the brain of the patient if the processing unit determines that the patient should undergo the scan of the brain.

In an example, the processing unit is configured to determine a degree of neurological impairment of the patient comprising utilization of the plurality of first task trial results and the scan of the brain of the patient.

In other words, variability in carrying out trials of task a number of times can be used to indicate that the patient should have a scan such as an MRI scan or PET scan or other scan. Then the scan can be assessed along with the variability in the trials of the task in determining a degree of cognitive impairment.

In an example, the image acquisition unit is an MRI image acquisition unit. The task interaction unit is configured to present a plurality of the trials of the first task to the patient during the MRI scan of the brain of the patient and acquire a plurality of repeated first task trial results generated by the patient having conducted the plurality of trials of the first task during the MRI scan of the brain of the patient. The processing unit is configured to determine a degree of neurological impairment of the patient comprising utilization of the plurality of repeated first task trial results and the MRI scan of the brain of the patient.

In this manner, in effect functional MRI (fMRI) can be undertaken in a completely new way. Variability in how a patient conducts trials of a same task can be used to determine if the patient needs an MRI scan. Then the patient during the MRI scan can carry out trials of the task a number of times again in order that the MRI scan can better establish what the problem is with respect to the abnormal variability in the patient carrying out the task. In other words, it is determined that the patient should have an MRI scan and an fMRI scan is taken, where the patient in effect repeats the trials of the task that they initially performed during the fMRI scan

In a third aspect, there is provided a cognitive impairment determination method. The method comprises:
providing a processing unit with a plurality of first task trial results, wherein the plurality of first task trial results is generated by a patient conducting a plurality of trials of a first task;
determining by the processing unit an index for the patient, wherein the determining the index comprises calculating of a representation of fluctuations in the plurality of first task trial results;
determining by the processing unit that the patient should undergo a scan of the brain, wherein the determining comprises utilizing the index for the patient; and
outputting information that the patient should undergo the scan of the brain if the processing unit determines that the patient should undergo the scan of the brain.

In a fourth aspect, there is provided a cognitive impairment determination method. The method comprises:
presenting by a task interaction unit a plurality of trials of a first task to a patient;
acquiring by the task interaction unit a plurality of first task trial results generated by the patient conducting the plurality of trials of the first task;
providing by the task interaction unit the plurality of first task trial results to a processing unit;
determining by the processing unit an index for the patient, wherein the determining the index comprises calculating a representation of fluctuations in the plurality of first task trial results;
determining by the processing unit that the patient should undergo a scan of the brain, wherein the determining comprises utilizing the index for the patient; and
carrying out by an image acquisition unit a scan of the brain of the patient if the processing unit determines that the patient should undergo the scan of the brain.

According to another aspect, there is provided a computer program element controlling one or more of the apparatuses and/or systems as previously described which, if the computer program element is executed by a processor, is adapted to perform the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic example of a cognitive impairment determination apparatus;
Fig. 2 shows a schematic example of a cognitive impairment determination system;
Fig. 3 shows a schematic example of a cognitive impairment determination method; and
Fig. 4 shows a schematic example of a cognitive impairment determination method.

### DETAILED DESCRIPTION OF EMBODIMENTS

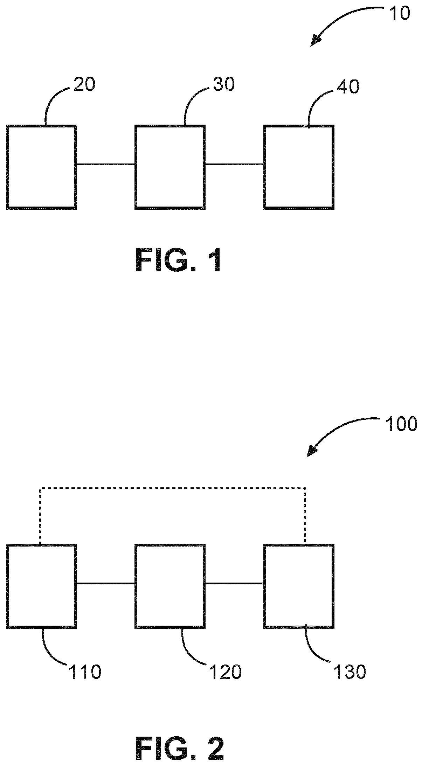

Fig. 1 shows an example of a cognitive impairment determination apparatus 10. The apparatus 10 comprises an input unit 20, a processing unit 30, 120, and an output unit 40. The input unit is configured to provide the processing unit with a plurality of first task trial results. The plurality of first task trial results is generated by a patient having conducted a plurality of trials of a first task. The processing unit is configured to determine an index for the patient. The determination of the index comprises a calculation of a representation of fluctuations in the plurality of first task trial results. The processing unit is configured to determine that the patient should undergo a scan of the brain, and the determination comprises utilization of the index for the patient. The output unit is configured to output information that the patient should undergo the scan of the brain if the processing unit determines that the patient should undergo the scan of the brain.

In an example, the scan of the brain is an MRI scan.

In an example, the scan of the brain is a structural MRI scan.

In an example, the scan of the brain is a fMRI scan.

In an example, the scan of the brain is an ASL scan to assess blood flow.

In an example, the scan of the brain is a PET scan.

Here a task means something that the patient is asked to do a number of times over a number of trials. This could for example relate to an instructions for a reaction time task, where a patient is asked to press a button only when they see a red dot on a screen. This is the task, and the trial results can comprise the times between the red dot being shown and the time the patient pressed the button, providing a range in times used in determining the index. The task could also for example be to think of as many words as possible starting with the letter E in a 5 minute period. The trial result can then be the number of words the patient thought of. The patient could then be given this task on a periodic basis, for example daily, weekly or monthly, and the variability in the number of words starting with the letter E (the trial results) can be used to determine an index for the patient.

In an example, the MRI scan that may be determined to be performed can be a structural MRI, for example a diffusion scan to assess structural connectivity.

In an example, the MRI scan that may be determined to be performed can be an fMRI scan, where for example the patient repeats the trials of the task during the scan.

In an example, the MRI scan that may be determined to be performed can be an fMRI scan without trails of the task, for example for a resting state to investigate functional connectivity.

In an example, it is determined that no scan of the brain is required is required.

According to an example, each first task trial result of the plurality of first task trial results comprises a time for the patient to conduct the associated trial of the first task.

According to an example, the calculation of the representation of fluctuations in the plurality of first task trial results comprises calculation of a standard deviation associated with the plurality of first task trial results.

In an example, the first task trial results are segregated into a plurality of bins, and wherein the calculation of the representation of fluctuations in the plurality of first task trial results comprises a calculation of fluctuations in the first task trial results in each of the bins of the plurality of bins.

Thus, for example regarding the example of the patient being asked to press a button only when they see a red dot, the red dot could be shown 400 times. A first trial bin is the 1-100 times the red button is shown, and the second trial bin is the 101-200 times the red button is shown. The times taken for the patient to press the button is determined for each trial (when the red dot is shown) and information relating to the patient not pressing the button or incorrectly pressing the button - for example when a blue dot is shown can be logged. Then for example, in the first trial bin the patient could be very slow and makes many mistakes and in the second trial bin the patient is faster and hardly makes any mistakes. Thus, for example in the first 1-100 trials there could be 76% errors, and in the next 101-200 trials there could be 5 % errors etc, and this provides valuable information as to whether the patient should undergo a MRI scan. Thus, this variation can be used in determining if the patient has a neurological impairment.

Also, the example of a patient being asked to think of words beginning with E this can also be compartmentalized into bins and the variation across bins analysed. Thus, a 10 minute period could have five 2 minute bins. In the first 2 minutes the patient my think of many correct words, in the second 2 minutes the patient may think of only a few correct words, whilst in the third the patient may only think of incorrect words with an F etc, and this variation and omissions/error rate can inform the system that an MRI scan of the patient should be undertaken.

According to an example, each trial of the plurality of trials of the first task was conducted on the same day.

According to an example, at least one trial of the plurality of trials of the first task was conducted on a different day to another trial of the plurality of trials of the first task.

In an example, each trial of the plurality of trials of the first task was conducted on a different day to the other trials of the first task.

Thus, as explained above the task could be to think of a number of words beginning with E in 5 minutes, and this could be carried out periodically on different days to determine if there is neurological impairment. Also, the patient could carry out the task of pressing a button when a red dot is shown a number or timed (trials) on one day and then repeat the task a number of times (further trials) on a different day. The variability in the trial results between days, and indeed the variability of trial results with a day, can be used in determining if the patient should have an MRI scan of their brain.

According to an example, the input unit is configured to provide the processing unit with a plurality of second task trial results, wherein the plurality of second task trial results is generated by the patient having conducted a plurality of trials of a second task, different to the first task, and the determination of the index comprises a calculation of a representation of fluctuations in the plurality of second task trial results.

In an example, each second task trial result of the plurality of second task trial results comprises a time for the patient to conduct the associated trial of the second task.

In an example, the calculation of the representation of fluctuations in the plurality of second task trial results comprises calculation of a standard deviation associated with the plurality of second task trial results.

In an example, each trial of the plurality of trials of the second task was conducted on the same day.

In an example, at least one trial of the plurality of trials of the second task was conducted on a different day to another trial of the plurality of trials of the second task.

In an example, each trial of the plurality of trials of the second task was conducted on a different day to the other trials of the second task.

In an example, the second task trial results are segregated into a plurality of bins, and wherein the calculation of the representation of fluctuations in the plurality of second task trial results comprises a calculation of fluctuations in the second task trial results in each of the bins of the plurality of bins.

According to an example, determination of the index comprises utilization of a first weighting factor applied to the representation of fluctuations in the plurality of first task trial results and a second weighting factor applied to the representation of fluctuations in the plurality of second task trial results.

According to an example, the determination that the patient should undergo the scan of the brain comprises a comparison of the index for the patient with a threshold index value.

According to an example, the threshold is determined from normalization tables and/or expert input.

Fig. 2 shows an example of a cognitive impairment determination system 100. The system 100 comprises a task interaction unit 110, a processing unit 30, 120, and an image acquisition unit 130. The task interaction unit is configured to present a plurality of trials of a first task to a patient and acquire a plurality of first task trial results generated by the patient having conducted the plurality of trials of the first task. The task interaction unit is configured to provide the plurality of first task trial results to the processing unit. The processing unit is configured to determine an index for the patient. The determination of the index comprises a calculation of a representation of fluctuations in the plurality of first task trial results. The processing unit is configured to determine that the patient should undergo a scan of the brain, and the determination comprises utilization of the index for the patient. The image acquisition unit is configured to carry out a scan of the brain of the patient if the processing unit determines that the patient should undergo the scan of the brain.

In an example, the image acquisition unit is an MRI image acquisition unit.

In an example, the image acquisition unit is a structural MRI image acquisition unit.

In an example, the image acquisition unit is a fMRI image acquisition unit.

In an example, the image acquisition unit is an ASL image acquisition unit.

In an example, the image acquisition unit is an PET image acquisition unit.

In an example, the scan of the brain is an MRI scan.

In an example, the scan of the brain is a structural MRI scan.

In an example, the scan of the brain is a fMRI scan.

In an example, the scan of the brain is an ASL scan to assess blood flow.

In an example, the scan of the brain is a PET scan.

According to an example, the processing unit is configured to determine a degree of neurological impairment of the patient comprising utilization of the plurality of first task trial results and the scan of the brain of the patient.

According to an example, the image acquisition unit is an MRI image acquisition unit. The task interaction unit is configured to present a plurality of the trials of the first task to the patient during the MRI scan of the brain of the patient and acquire a plurality of repeated first task trial results generated by the patient having conducted the plurality of trials of the first task during the MRI scan of the brain of the patient. The processing unit is configured to determine a degree of neurological impairment of the patient comprising utilization of the plurality of repeated first task trial results and the MRI scan of the brain of the patient.

In an example, each repeated first task trial result of the plurality of repeated first task trial results comprises a time for the patient to conduct the associated trial of the first task during the scan.

In an example, the calculation of the representation of fluctuations in the plurality of repeated first task trial results comprises calculation of a standard deviation associated with the plurality of repeated first task trial results.

In an example, the repeated first task trial results are segregated into a plurality of bins. The calculation of the representation of fluctuations in the plurality of repeated first task trial results comprises a calculation of fluctuations in the repeated first task trial results in each of the bins of the plurality of bins.

In an example, each trial of the plurality of trials of the first task was conducted on the same day.

In an example, at least one trial of the plurality of trials of the first task was conducted on a different day to another trial of the plurality of trials of the first task.

In an example, each trial of the plurality of trials of the first task was conducted on a different day to the other trials of the first task.

In an example, the task interaction unit is configured to present a plurality of trials of a second task to the patient and acquire a plurality of second task trial results generated by the patient having conducted the plurality of trials of the second tasks, wherein the second task is different to the first task, and wherein the determination of the index comprises a calculation of a representation of fluctuations in the plurality of second task trial results.

In an example, each second task trial result of the plurality of second task trial results comprises a time for the patient to conduct the associated trial of the second task.

In an example, the calculation of the representation of fluctuations in the plurality of second task trial results comprises calculation of a standard deviation associated with the plurality of second task trial results.

In an example, the second task trial results are segregated into a plurality of bins. The calculation of the representation of fluctuations in the plurality of second task trial results comprises a calculation of fluctuations in the second task trial results in each of the bins of the plurality of bins.

In an example, each trial of the plurality of trials of the second task was conducted on the same day.

In an example, at least one trial of the plurality of trials of the second task was conducted on a different day to another trial of the plurality of trials of the second task.

In an example, each trial of the plurality of trials of the second task was conducted on a different day to the other trials of the second task.

In an example, determination of the index comprises utilization of a first weighting factor applied to the representation of fluctuations in the plurality of first task trial results and a second weighting factor applied to the representation of fluctuations in the plurality of second task trial results.

In an example, the determination that the patient should undergo the scan such as an MRI scan or PET scan or other scan of the brain comprises a comparison of the index for the patient with a threshold index value.

In an example, the threshold is determined from normalization tables and/or expert input.

In an example, the processing unit is configured to determine a degree of neurological impairment of the patient comprising utilization of the plurality of second task trial results and the scan of the brain of the patient.

In an example, the task interaction unit is configured to present a plurality of trials of the second task to the patient during the scan of the brain of the patient and acquire a plurality of repeated second task trial results generated by the patient having conducted the plurality of trials of the second task during the MRI scan of the brain of the patient. The processing unit is configured to determine a degree of neurological impairment of the patient comprising utilization of the plurality of repeated second task trial results and the MRI scan of the brain of the patient.

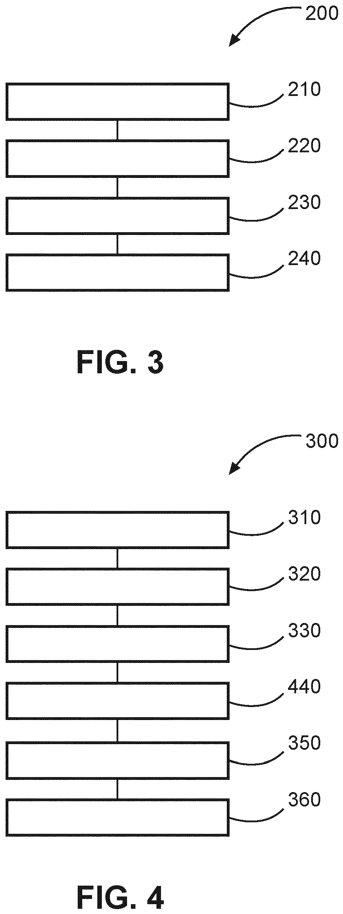

Fig. 3 shows a cognitive impairment determination method 200 in its basic steps. the method 200 comprises:
providing 210 a processing unit with a plurality of first task trial results, wherein the plurality of first task trial results is generated by a patient conducting a plurality of trials of a first task;
determining 220 by the processing unit an index for the patient, wherein the determining the index comprises calculating of a representation of fluctuations in the plurality of first task trial results; determining 230 by the processing unit that the patient should undergo a scan of the brain, wherein the determining comprises utilizing the index for the patient; and
outputting 240 information that the patient should undergo the scan of the brain if the processing unit determines that the patient should undergo the scan of the brain.

In an example, the scan of the brain is an MRI scan.

In an example, the scan of the brain is a structural MRI scan.

In an example, the scan of the brain is a fMRI scan.

In an example, the scan of the brain is an ASL scan to assess blood flow.

In an example, the scan of the brain is a PET scan.

In an example, each first task trial result of the plurality of first task trial results comprises a time for the patient to conduct the associated trial of the first task.

In an example, the calculating the representation of fluctuations in the plurality of first task trial results comprises calculating a standard deviation associated with the plurality of first task trial results.

In an example, the first task trial results are segregated into a plurality of bins, and wherein the calculation of the representation of fluctuations in the plurality of first task trial results comprises a calculation of fluctuations in the first task trial results in each of the bins of the plurality of bins.

In an example, each trial of the plurality of trials of the first task was conducted on the same day.

In an example, at least one trial of the plurality of trials of the first task was conducted on a different day to another trial of the plurality of trials of the first task.

In an example, each trial of the plurality of trials of the first task was conducted on a different day to the other trials of the first task.

In an example, the method comprises providing the processing unit with a plurality of second task trial results, and the plurality of second task trial results is generated by the patient conducting a plurality of trials of a second task, different to the first task, and the determining the index comprises calculating a representation of fluctuations in the plurality of second task trial results.

In an example, each second task trial result of the plurality of second task trial results comprises a time for the patient to conduct the associated trial of the second task.

In an example, the calculating the representation of fluctuations in the plurality of second task trial results comprises calculating a standard deviation associated with the plurality of second task trial results.

In an example, each trial of the plurality of trials of the second task was conducted on the same day.

In an example, at least one trial of the plurality of trials of the second task was conducted on a different day to another trial of the plurality of trials of the second task.

In an example, each trial of the plurality of trials of the second task was conducted on a different day to the other trials of the second task.

In an example, the second task trial results are segregated into a plurality of bins, and the calculation of the representation of fluctuations in the plurality of second task trial results comprises a calculation of fluctuations in the second task trial results in each of the bins of the plurality of bins.

In an example, the determining the index comprises applying a first weighting factor to the representation of fluctuations in the plurality of first task trial results and a applying second weighting factor to the representation of fluctuations in the plurality of second task trial results.

In an example, the determining that the patient should undergo the MRI scan of the brain comprises comparing the index for the patient with a threshold index value.

In an example, the threshold is determined from normalization tables and/or expert input.

Fig. 4 shows a cognitive impairment determination method 300 in its basic steps. The method 300 comprises:
presenting 310 by a task interaction unit a plurality of trials of a first task to a patient;
acquiring 320 by the task interaction unit a plurality of first task trial results generated by the patient conducting the plurality of trials of the first task;
providing 330 by the task interaction unit the plurality of first task trial results to a processing unit;
determining 340 by the processing unit an index for the patient, wherein the determining the index comprises calculating a representation of fluctuations in the plurality of first task trial results;
determining 350 by the processing unit that the patient should undergo a scan of the brain, wherein the determining comprises utilizing the index for the patient; and
carrying 360 out by an image acquisition unit a scan of the brain of the patient if the processing unit determines that the patient should undergo the scan of the brain.

In an example, the scan of the brain is an MRI scan.

In an example, the scan of the brain is a structural MRI scan.

In an example, the scan of the brain is a fMRI scan.

In an example, the scan of the brain is an ASL scan to assess blood flow.

In an example, the scan of the brain is a PET scan.

In an example, the method comprises determining by the processing unit a degree of neurological impairment of the patient comprising utilizing the plurality of first task trial results and the scan of the brain of the patient.

In an example, the image acquisition unit is an MRI image acquisition unit, and the method comprises presenting by the task interaction unit a plurality of trials of the first task to the patient during the MRI scan of the brain of the patient and acquiring a plurality of repeated first task trial results generated by the patient conducting the plurality of trials of the first task during the MRI scan of the brain of the patient, and determining by the processing unit a degree of neurological impairment of the patient comprising utilizing the plurality of repeated first task trial results and the MRI scan of the brain of the patient.

In an example, each repeated first task trial result of the plurality of repeated first task trial results comprises a time for the patient to conduct the associated trial of the first task.

In an example, the calculating the representation of fluctuations in the plurality of repeated first task trial results comprises calculating a standard deviation associated with the plurality of repeated first task trial results.

In an example, the repeated first task trial results are segregated into a plurality of bins, and the calculation of the representation of fluctuations in the plurality of repeated first task trial results comprises a calculation of fluctuations in the repeated first task trial results in each of the bins of the plurality of bins.

In an example, each trial of the plurality of trials of the first task was conducted on the same day.

In an example, at least one trial of the plurality of trials of the first task was conducted on a different day to another trial of the plurality of trials of the first task.

In an example, each trial of the plurality of trials of the first task was conducted on a different day to the other trials of the first task.

In an example, the method comprises presenting by the task interaction unit a plurality of trials of a second task to the patient and acquiring a plurality of second task trial results generated by the patient conducting the plurality of trials of the second task. The second task is different to the first task, and the determining of the index comprises calculating a representation of fluctuations in the plurality of second task trial results.

In an example, each second task trial result of the plurality of second task trial results comprises a time for the patient to conduct the associated trial of the second task.

In an example, the calculating the representation of fluctuations in the plurality of second task trial results comprises calculating a standard deviation associated with the plurality of second task trial results.

In an example, the second task trial results are segregated into a plurality of bins, and wherein the calculation of the representation of fluctuations in the plurality of second task trial results comprises a calculation of fluctuations in the second task trial results in each of the bins of the plurality of bins.

In an example, each trial of the plurality of trials of the second task was conducted on the same day.

In an example, at least one trial of the plurality of trials of the second task was conducted on a different day to another trial of the plurality of trials of the second task.

In an example, each trial of the plurality of trials of the second task was conducted on a different day to the other trials of the second task.

In an example, the determining the index comprises applying a first weighting factor to the representation of fluctuations in the plurality of first task trial results and applying a second weighting factor to the representation of fluctuations in the plurality of second task trial results.

In an example, the determining that the patient should undergo the MRI scan of the brain comprises comparing the index for the patient with a threshold index value.

In an example, the threshold is determined from normalization tables and/or expert input.

In an example, the method comprises determining by processing unit a degree of neurological impairment of the patient comprising utilizing the plurality of second task trial results and the MRI scan of the brain of the patient.

In an example, the image acquisition unit is an MRI image acquisition unit, and the method comprises presenting by the task interaction unit a plurality of trials of the second task to the patient during the MRI scan of the brain of the patient and acquiring a plurality of repeated second task trial results generated by the patient conducting the plurality of trials of the second task during the MRI scan of the brain of the patient, and determining by the processing unit a degree of neurological impairment of the patient comprising utilizing the plurality of repeated second task trial results and the MRI scan of the brain.

Thus, the new technique makes use of information on trial-by-trial fluctuations in performance. Large fluctuations are indicative of impaired connectivity, which likely results from diffuse axonal injury, the primary neurological damage in mTBI. In the new technique digital cognitive testing has provided the opportunity to study test outcomes and generate such a measure of fluctuations in performance, which has been found to be eminently suitable to detect subtle cognitive deficits characteristic of mTBI. This then provides a gatepost to indicate whether there is a clinical need to conduct MRI in a particular patient. High intra-individual variability was found to be typical for patients with subtle cognitive impairment. As opposed to averaged test scores, variability measures have been demonstrated to correlate with subjective reports of dysfunction in daily life (see for example Spreij, L. In: Neuropsychology from paper-and-pencil to technology. Advancing cognitive rehabilitation. Chapter 4: The journey is just as important as the destination - Digital neuropsychological assessment provides performance stability measures, p. 101. 2020), and this variability has been utilized in a new way to determine if a patient should have an MRI scan.

The new technique utilizes trial-by-trial fluctuations in performance on cognitive tasks to provide clear indications of subtle cognitive impairment. This then in effect provides for a red flag to be generated, signalling that further diagnostic imaging of underlying brain abnormalities is required, in particular regarding impaired functional and/or structural connectivity through an MRI scan or other advanced scan such as ASL or PET. Thus when a patient shows signs of cognitive impairment, via the new technique, the use of advanced imaging techniques (MRI/ASL/PET) that provide insight into the subtle microstructural, microvascular and functional brain damage typical of mTBI can be utilized, where such damage cannot be diagnosed with conventional tools (such as CT).

Demonstrating brain damage in mTBI with advanced (e,g, MRI/ASL/PET) imaging tools through the new technique is important because it improves diagnosis and enables a timely start of suitable treatment programs. A more accurate and timely diagnosis enables patients to better manage their (in some cases, temporary) impairment and will likely lead to quicker recovery and less impact on quality of life. Early intervention is known to lead to significant cost savings (>USD 2000 per patient per year) (see: Leibson, C.L., Brown, A.W., Hall Long, K., Ransom, J.E., Mandrekar, J., Osler, T.M., & Malec, J.F. (2012). Medical care costs associated with traumatic brain injury over the full spectrum of disease: a controlled population-based study. Journal of Neurotrauma, 29(11), 2038-2049, and Griesbach, G.S., Kreber, L.A., Harrington, D., & Ashley, M.J. (2015). Post-acute brain injury rehabilitation on outcome measures and life care costs. Journal of Neurotrauma, 32, 704-711). It is to be noted that the new technique described here is not only relevant for mTBI. It is applicable to a variety of patients suffering from subtle cognitive impairment, such as those with early signs of neurodegeneration (MS, dementia).

In summary, the new technique overcomes at least the following:
Current tests that are conducted in order to reveal cognitive dysfunction in patients with mTBI are not sufficiently sensitive to detect subtle, but serious cognitive deficits that impair normal everyday functioning. This results in suboptimal treatment and unnecessary healthcare costs later in the treatment process. The new technique can detect such subtle, but serious cognitive deficits enabling a follow-on advanced imaging scan (e.g. a structural MRI scan, a fMRI scan, a ASL scan, a PET scan) to be carried out.

Literature states that specifically for brain injury patients with mild cognitive deficits it is important to conduct an advanced brain scan (e.g. MRI, ASL, PET) because acute as well as longer-term functional outcome in mTBI appear to be associated with white matter changes detectable for example on advanced MRI (see: Richter S, Winzeck S, Kornaropoulos EN, Das T, Vande Vyvere T, Verheyden J, Williams GB, Correia MM, Menon DK, Newcombe VFJ; Neuroanatomical Substrates and Symptoms Associated With Magnetic Resonance Imaging of Patients With Mild Traumatic Brain Injury. JAMA Netw Open. 2021 Mar 1;4(3):e210994). The new technique enables it to be determined to carry out such imaging that can, thus, document the evolution of pathology, thereby highlighting windows for therapy. In addition, the advanced scan (such as MRI, ASL, PET) to be determined to be utilized for the patient by the new technique can provide prognostic information to help select patients for clinical follow-up or interventional trials.

As described above, currently, advanced brain scans (such as MRI, ASL, PET) are not routinely conducted because taking an MRI/ASL/PET is relatively expensive and not necessary for all mTBI patients. Furthermore, focussing here on MRI, if MRI scans are made, it usually involves standard MRI scanning (T1/T2), which is not sufficiently suited to detect the subtle microstructural, microvascular and functional brain damage typical of mTBI. Proper diagnosis requires the use of more advanced scanning techniques, such as Diffusion Tensor Imaging (DTI), Myeline Water Imaging (MWI), Susceptibility-weighted Imaging (SWI), and functional MRI (fMRI). The new technique enables test results from a patient to be analysed to determine a signal that such advanced MRI techniques should be instituted, or indeer other imaging modalities such as ASL/PET.

In summary the new technique provides a first step of conducting a cognitive test to determine if an advanced scan (MRI/ASL/PET) should be undertaken, followed by a second step of undertaking advanced scanning if necessary.

In the following discussion the imaging modality relates to MRI for simplicity of explanation, but it is to be understood that other advance imaging modalities such as ASL and PET could be utilized.

In an expanded summary these steps are:
First-Cognitive test: The new technique increases the sensitivity of cognitive tests by involving an analysis of trial-by-trial fluctuations in cognitive test performance as opposed to current analysis of average performance across multiple trials. Such a first step, also termed an initial sensitive pre-MR test, can be taken asap after the injury to the patient when the patient is able to do so, to first select patients who would benefit from receiving an MR brain scan. This sensitive test involves a cognitive task, e.g. a reaction time task designed to assess attention. The task consists of multiple trials and / or is undertaken a number of times and performance fluctuations across these trials is calculated. However, multiple different cognitive tasks can be provided to the patient with sufficient repetitions (trials) of each single cognitive task. In this way instability across tasks instead of within one single task can be examined. A data processing unit automatically provides a cognitive instability index, reflecting trial-by-trial fluctuations in performance for the or each task. In case the index exceeds a predetermined threshold, an automatic flag signal, with an attached level of urgency, is provided and an MRI scan is scheduled accordingly. The threshold can be based on norm scores. It is also to be noted that the task in its multiple trials can be taken by the patient over an extended period of time. Here, the results could then be used to determine if the patient appears to have a cognitive impairment for example from MS or dementia, and again an appropriate MRI scan could be instituted to obtain further information.
Second - MR scanning: Upon selection of the patient to have an MRI scan, for example it has been determined that they may have mTBI, a technical device (an MR software module) can be used to provide patients with a quantitative (biomarker-)index that correlates trial-by-trial variability in cognitive performance with (variation in) activity of a particular brain area or neuronal networks and/or with measures of structural and/or functional connectivity.

The complete new technique involving imaging via advanced MRI techniques is discussed in detail below. It is to be noted that a pared version new technique can be just to determine that a patient should have an MRI scan, without actually carrying out that scan.

Thus, the building blocks of the complete new technique can be summarised as comprising:
**A. Cognitive Data Input Unit:** Information is collected with (digital) cognitive tests. An index of cognitive instability is derived.
**B. Flag Unit:** This index may or may not flag the clinical need for conducting (advanced) imaging (MRI/ASL/PET).
**C. Imaging Data Input Unit:** If indeed flagged, measures of functional and structural connectivity are collected with advanced imaging techniques (MRI/ASL/PET).
**D. Integration Unit:** Data from A. and C. are combined to determine the degree to which impaired connectivity causes mTBI symptomatology in a particular patient.
**E. Presentation Unit:** Results and recommendations are presented on a digital dashboard.

These units are now discussed in specific detail:

### A-Cognitive data input unit:

Cognitive data is collected during a (digital) assessment that includes tests that are specifically developed to measure impairments typical for mTBI patients, such as deficits in attention, memory, processing speed and executive functioning. The cognitive tests are designed in such a way that they include multiple trials, e.g. tests in which the patient must react as quickly as possible to task-relevant information while ignoring or suppressing reactions to distracting information. Examples of suitable cognitive tests are vigilance tasks (such as the Continuous Performance Task), the Flanker task, the Posner cueing task, or the Stroop task. Cognitive data collected can include standard outcome measures such as errors, omissions, and reaction time, but more importantly, includes measures of intra-individual variability in performance, such as the standard deviation of reaction times to stimuli on trials presented during certain pre-defined time bins (see for example: Spreij, L. In: Neuropsychology from paper-and-pencil to technology. Advancing cognitive rehabilitation. Chapter 4: The journey is just as important as the destination - Digital neuropsychological assessment provides performance stability measures, p. 101. 2020). This information is used to calculate an index for cognitive instability. To explain further, standard deviation of reaction times can be assessed that are collected from one single task with multiple trails. In other words, all trials are one bin. However, multiple trials from a single task can be divided into separate bins. For example for a task that has 400 trials these can be divided into 8 bins with 50 trials. This way other measures reflecting fluctuations, e.g. from the error or omission rates across bins can be calculated.

### B-Flag Unit:

If the cognitive instability index exceeds a pre-defined threshold, it is considered indicative of neurological damage, and advanced imaging scanning (e.g. MRI/ASL/PET) is recommended for a particular patient.

### C- Imaging Data Input Unit:

Imaging data that provides information on functional and structural connectivity is collected. The use of advanced neuroimaging techniques that are suited to detect the subtle microstructural (DTI, MWI), microvascular (SWI) and functional (fMRI) brain damage typical of mTBI is important. Connectivity indices are derived.

### D-Integration Unit:

Outcomes of the 'cognitive data input unit' and the 'imaging data input unit' are combined. Information on functional and structural network status reveals which brain abnormalities underlie cognitive instability, as well as other mTBI symptoms.

### E-Presentation Unit:

This can be a dashboard with automatic presentation of integrated information. The presentation unit can have several steps. First, information from the cognitive tests can be presented along with information about whether - based on the trial-by-trial variability of a given patient- there is a clinical need for an advanced scan (MRI/fMRI/ASL/PET etc). Second, information from the advanced scan (MRI/fMRI/ASL/PET etc) can be presented along with an integrated presentation of the cognitive and imaging data. Patients whose performance on cognitive tests exceeds the pre-defined threshold for cognitive instability are more likely to have neurological damage that may result in chronic deficits. Imaging data can inform whether patients are indeed likely to experience chronic deficits thereby providing a more accurate diagnosis. This enables a more specific diagnosis of mTBI which allows for a personalized treatment plan. Currently, rehabilitation treatment is largely organized as 'one size fits all'. In a third step, information about prognosis and a personalized treatment plan can be presented. This dashboard with integrated information improves communication between different healthcare experts and across the care pathway (acute vs chronic). Data can be shared on this platform.

Details on the further embodiments is now described:
The cognitive instability index described above (for example immediately after injury) reflects performance fluctuations during a particular task. It could also be calculated differently - in a way that reflects performance fluctuations across cognitive tasks or over time (e.g. weeks, months, or years), which opens up the opportunity to monitor changes over time and assess treatment efficacy. Thus, rather than having multiple trials within a single task, fluctuations across different tasks (instability in the memory domain vs attention domain etc) can be compared or trials can be at different points in time (right after brain injury vs 3 months post-injury). The latter enable longitudinal monitoring of cognitive instability.

Cognitive instability can be monitored for example as follows: For each example out of a set of multiple tasks and/or multiple time points (n), a measure of cognitive instability (e.g. standard deviation of reaction time, SDRT) is calculated. This results in [SDa, SDb,.... SDn], where SDa-n are standard deviations of reaction time for different tasks and/or for a task at different time points. By weighting these separate measures a (sub)index can be calculated additionally: e.g. InstabilityIndex = x times SDa + y times SDb, +... z times SDn. With xyz being predetermined weighting factors.

The use of advanced imaging techniques provides insight into brain mechanisms underlying fluctuations in performance, i.e. impaired structural connectivity (diffuse axonal injury, myelin damage) and/or impaired functional connectivity. Cognitive tests can also be presented during (rather than before) scanning. Thus task related fMRI can be conducted, where cognitive trial results relating to a task the patient is undertaking during MRI is in effect integrated into the MRI data. This enables an determination of what activity in what brain areas co-occurs in time with what performance (in this case performance fluctuations). This allows further investigation of the exact mechanism underlying cognitive instability in a particular patient, e.g.
*Intra-individual variation can be linked to fluctuating activity - or total degree of activity - in the superordinate network for cognitive control, comprising the dorsal regions of the lateral prefrontal cortex (DLPFC), anterior cingulate cortex (dACC) and parietalcortex (DPC).
*Fluctuations in task performance can be linked to (dynamic fluctuations in) activity in the Default Mode Network relative to the Executive Control Network, reflecting the degree to which a particular patient is able to focus solely on the task requirements.
*Fluctuations in task performance can be linked to the degree to which patients selectively activate brain areas that are optimally suited for task completion and/or reflect deployment of compensation mechanisms.
*Trial-by-trial analysis of fMRI data can reveal fluctuations in neuronal activity in pre-defined brain areas (e.g. precuneus).

Information presented on the dashboard can be linked to other information, such as demographics, medical history, comorbid disorders etc.

Information presented on the dashboard can also be linked to a longer term clinical outcome, which would - over time - lead to better prognosis. Successful prediction of who is likely to recover and who is not likely to recover, enables early intervention in those patients that benefit most from treatment.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A cognitive impairment determination apparatus (10), comprising:
- an input unit (20);
- a processing unit (30, 120); and
- an output unit (40);
wherein the input unit is configured to provide the processing unit with a plurality of first task trial results, wherein the plurality of first task trial results is generated by a patient having conducted a plurality of trials of a first task;
wherein the processing unit is configured to determine an index for the patient, wherein the determination of the index comprises a calculation of a representation of fluctuations in the plurality of first task trial results;
wherein the processing unit is configured to determine that the patient should undergo a scan of the brain, wherein the determination comprises utilization of the index for the patient; and
wherein the output unit is configured to output information that the patient should undergo the scan of the brain if the processing unit determines that the patient should undergo the scan of the brain.

2. Apparatus according to claim 1, wherein each first task trial result of the plurality of first task trial results comprises a time for the patient to conduct the associated trial of the first task.

3. Apparatus according to any of claims 1-2, wherein the calculation of the representation of fluctuations in the plurality of first task trial results comprises calculation of a standard deviation associated with the plurality of first task trial results.

4. Apparatus according to any of claims 1-3, wherein each trial of the plurality of trials of the first task was conducted on the same day.

5. Apparatus according to any of claims 1-3, wherein at least one trial of the plurality of trials of the first task was conducted on a different day to another trial of the plurality of trials of the first task.

6. Apparatus according to any of claims 1-5, wherein the input unit is configured to provide the processing unit with a plurality of second task trial results, wherein the plurality of second task trial results is generated by the patient having conducted a plurality of trials of a second task, different to the first task, and wherein the determination of the index comprises a calculation of a representation of fluctuations in the plurality of second task trial results.

7. Apparatus according to claim 6, wherein determination of the index comprises utilization of a first weighting factor applied to the representation of fluctuations in the plurality of first task trial results and a second weighting factor applied to the representation of fluctuations in the plurality of second task trial results.

8. Apparatus according to any of claims 1-7, wherein the determination that the patient should undergo the scan of the brain comprises a comparison of the index for the patient with a threshold index value.

9. Apparatus according to claim 8, wherein the threshold is determined from normalization tables and/or expert input.

10. A cognitive impairment determination system (100), comprising:
- a task interaction unit (110);
- a processing unit (30, 120); and
- an image acquisition unit (130);
wherein the task interaction unit is configured to present a plurality of trials of a first task to a patient and acquire a plurality of first task trial results generated by the patient having conducted the plurality of trials of the first task;
wherein the task interaction unit is configured to provide the plurality of first task trial results to the processing unit;
wherein the processing unit is configured to determine an index for the patient, wherein the determination of the index comprises a calculation of a representation of fluctuations in the plurality of first task trial results;
wherein the processing unit is configured to determine that the patient should undergo a scan of the brain, wherein the determination comprises utilization of the index for the patient; and
wherein the image acquisition unit is configured to carry out a scan of the brain of the patient if the processing unit determines that the patient should undergo the scan of the brain.

11. System according to claim 10, wherein the processing unit is configured to determine a degree of neurological impairment of the patient comprising utilization of the plurality of first task trial results and the scan of the brain of the patient.

12. System according to any of claims 10-11, wherein the image acquisition unit is an MRI image acquisition unit, wherein the task interaction unit is configured to present a plurality of the trials of the first task to the patient during the MRI scan of the brain of the patient and acquire a plurality of repeated first task trial results generated by the patient having conducted the plurality of trials of the first task during the MRI scan of the brain of the patient, and wherein the processing unit is configured to determine a degree of neurological impairment of the patient comprising utilization of the plurality of repeated first task trial results and the MRI scan of the brain of the patient.

13. A cognitive impairment determination method (200), comprising:
providing (210) a processing unit with a plurality of first task trial results, wherein the plurality of first task trial results is generated by a patient conducting a plurality of trials of a first task;
determining (220) by the processing unit an index for the patient, wherein the determining the index comprises calculating of a representation of fluctuations in the plurality of first task trial results;
determining (230) by the processing unit that the patient should undergo a scan of the brain, wherein the determining comprises utilizing the index for the patient; and
outputting (240) information that the patient should undergo the scan of the brain if the processing unit determines that the patient should undergo the scan of the brain.

14. A cognitive impairment determination method (300), comprising:
presenting (310) by a task interaction unit a plurality of trials of a first task to a patient;
acquiring (320) by the task interaction unit a plurality of first task trial results generated by the patient conducting the plurality of trials of the first task;
providing (330) by the task interaction unit the plurality of first task trial results to a processing unit;
determining (340) by the processing unit an index for the patient, wherein the determining the index comprises calculating a representation of fluctuations in the plurality of first task trial results;
determining (350) by the processing unit that the patient should undergo a scan of the brain, wherein the determining comprises utilizing the index for the patient; and
carrying (360) out by an image acquisition unit a scan of the brain of the patient if the processing unit determines that the patient should undergo the scan of the brain.

15. A computer program element for controlling an apparatus according to any of claims 1-9 which when executed by a processor is configured to carry out the method of claim 13 and/or for controlling a system according to any of claims 10-12 which when executed by a processor is configured to carry out the method of claim 14.
